# DEMANDE DE BREVET EUROPEEN

(11) **EP 0 820 765 A2**
(43) Date de publication de la demande: **28.01.1998**
(21) Numéro de dépôt: 97401559.6
(22) Date de dépôt: 02.07.1997
(51) Int. Cl.: A61K 7/48

(54) **Utilisation du miel comme agent kératolytique**

(30) Priorité: 26.07.1996 FR 9609458
(71) Demandeur: L'OREAL, 75008 Paris (FR)
(72) Inventeur: Marion, Catherine, 92330 Sceaux (FR); Vanstraceele, Anne, 75013 Paris (FR)
(74) Mandataire: Tezier Herman, Béatrice

(57) **Abrégé**

L'invention se rapporte à l'utilisation du miel comme agent kératolytique dans une composition cosmétique et/ou dermatologique, notamment comme actif pour améliorer l'éclat du teint et/ou pour lisser la peau du visage et/ou du corps et/ou pour traiter les rides et les ridules.

L'utilisation du miel permet de lutter en douceur contre le vieillissement de la peau du visage et/ou du corps humain.

Selon une forme particulière de réalisation de l'invention, la composition selon l'invention contient en outre au moins un hydroxyacide et est tamponnée à pH 5.

## Description

L'invention se rapporte à l'utilisation du miel comme agent kératolytique dans une composition cosmétique ou dermatologique, notamment comme actif pour améliorer l'éclat du teint et/ou pour lisser la peau du visage et/ou du corps humain et/ou pour traiter les rides et les ridules. Elle se rapporte également à une composition topique contenant du miel et au moins un alcool gras. Elle se rapporte également à une composition topique contenant du miel et au moins un hydroxyacide, tamponnée à pH 5 et permettant de lutter en douceur contre le vieillissement de la peau du visage et/ou du corps humain.

On cherche, de plus en plus, à paraître plus jeune et moins ridé, en utilisant des compositions cosmétiques contenant des actifs capables de lutter contre le vieillissement.

Malheureusement, les actifs antivieillissement couramment utilisés tels que les rétinoïdes et les actifs acides comme les hydroxyacides, présentent l'inconvénient majeur de provoquer des picotements, des démangeaisons, des tiraillements après leur application, pouvant conduire à un important inconfort. L'utilisation de ces composés pour les utilisateurs à peau sensible est donc souvent rédhibitoire.

Pour pallier à ces inconvénients, on cherche à utiliser des hydroxyacides issus de produits naturels, tels que les fruits ou le miel. Ainsi, les α-hydroxyacides extraits du miel sont décrits comme étant beaucoup moins irritants que les produits de synthèse (voir Smith, Cosmetics & toiletries, Septembre 1994, vol. 109, p. 41-48). Ces acides sont obtenus par extraction de miel fermenté. Toutefois, ils présentent encore un certain inconfort.

Aussi, il subsiste le besoin d'une composition cosmétique et/ou dermatologique pour lutter contre le vieillissement, ne présentant pas ces inconvénients.

La demanderesse a trouvé de manière surprenante que le miel présentait des propriétés kératolytiques et permettait d'améliorer l'éclat du teint de la peau et de lisser les traits de la peau, faisant paraître ainsi la peau plus jeune et moins ridée.

Certes, il est connu d'utiliser le miel dans des compositions cosmétiques destinées à traiter le vieillissement. Ainsi, par exemple les documents JP-A-59-55809, GB-A-120673 et JP-A-6-38702 décrivent de telles compositions. Toutefois, dans ces documents, le miel est ajouté comme additif à des actifs agissant sur le vieillissement, et sa propre efficacité comme agent kératolytique n'a jamais été décrite.

Aussi, la présente invention a pour objet l'utilisation du miel dans une composition cosmétique, comme agent kératolytique et/ou comme actif pour améliorer l'éclat du teint et/ou pour lisser la peau et/ou pour traiter les rides et les ridules.

Elle a aussi pour objet l'utilisation du miel comme agent kératolytique et/ou comme actif pour améliorer l'éclat du teint et/ou pour lisser la peau et/ou pour traiter les rides et les ridules pour la préparation d'une composition dermatologique.

Le miel permet de lutter en douceur contre le vieillissement de la peau du visage et/ou du corps humain.

On entend par miel un miel non traité, c'est-à-dire non fermenté et ne contenant pas d'hydroxyacides, au contraire de l'extrait de miel qui est un produit de fermentation et qui contient des hydroxyacides.

Le miel utilisé selon l'invention se compose d'un mélange de glucose, levulose, dextrine, saccharose, protéines et eau, la teneur de ces différents constituants pouvant varier selon la provenance du miel. De manière classique, le miel comprend de 72 à 75 % du mélange de glucose et levulose, 15 % d'eau, 10 % de dextrine, 2,5 % de saccharose et 1 % de protéines.

Le miel peut avoir toute provenance. Il peut s'agir notamment de miels d'acacia, de tilleul, de montagne, de "gatinais", de fleurs d'oranger, de toutes fleurs.

La quantité de miel dans la composition selon l'invention doit être en une quantité efficace pour agir comme agent kératolytique et peut aller par exemple de 0,1 à 10 % et de préférence de 0,1 à 2 % du poids total de la composition.

Selon une forme particulière de réalisation de l'invention, la composition contient, en plus du miel, au moins un alcool gras permettant d'améliorer la stabilité de la composition.

Comme alcool gras, on peut notamment utiliser l'alcool cétylique et l'alcool stéarylique. La quantité d'alcool gras peut aller par exemple de 0,1 à 10 % et de préférence de 0,5 à 5 % du poids total de la composition.

Aussi, la présente invention a également pour objet une composition cosmétique ou dermatologique, caractérisée en ce qu'elle contient, dans un milieu cosmétiquement et/ou dermatologiquement acceptable, une quantité efficace de miel et au moins un alcool gras.

Selon une autre forme particulière de réalisation de l'invention, la composition contient, en plus du miel, un ou plusieurs actifs acides, et un tampon de pH 5. La demanderesse a, en effet, trouvé de manière surprenante qu'une telle composition présentait l'avantage de diminuer l'effet irritant des actifs acides sans diminuer l'activité de ces actifs. La présence simultanée du miel, d'actif acide et du tampon permet d'obtenir une composition ayant un rapport tolérance/efficacité particulièrement satisfaisant.

Aussi, la présente invention a également pour objet une composition cosmétique ou dermatologique, contenant dans un milieu cosmétiquement et/ou dermatologiquement acceptable, au moins un actif acide, caractérisée en ce qu'elle contient, en outre, du miel et un tampon de pH 5.

Comme actifs acides utilisables dans la composition de l'invention, on peut citer notamment les α-hydroxy-acides ou les β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxycarbonylés ou alcoxylés ayant de 2 à 18 atomes de carbone.

A titre d'α-hydroxy-acides, on peut citer les acides glycolique, lactique, malique, tartrique, citrique, mandélique. Comme β-hydroxy-acides, on peut citer l'acide salicylique ainsi que ses dérivés acylés, les acides hydroxy-2 alcanoïques et leurs dérivés, l'acide hydroxy-2 benzoîque et ses dérivés comme l'acide hydroxy-2-méthyl-3-benzoïque et l'acide hydroxy-2-méthoxy-3-benzoïque.

Comme dérivé de l'acide salicylique, on peut citer notamment ceux décrits dans les documents FR-A-2581542 et EP-A-378936, et notamment les acides n-octanoyl-5-salicylique, n-décanoyl-5-salicylique, n-dodécanoyl-5-salicylique, n-octyl-5-salicylique, n-heptyloxy-5-salicylique, n-heptyloxy4-salicylique. On peut aussi utiliser ceux décrits dans le document EP-A-570230.

On peut aussi utiliser comme actifs les α- ou β-céto-acides.

La quantité d'actif acide peut aller de 0,01 à 20 % et de préférence de 0,1 à 5 % du poids total de la composition.

Pour obtenir la composition tamponnée selon l'invention, on ajuste son pH à 5 à l'aide de bases, telles que la soude ou la triéthanolamine, puis on ajoute le tampon de pH 5.

Le tampon présent dans la composition selon l'invention peut être notamment un tampon citrate de pH 5. La quantité de tampon présent dans la composition peut aller par exemple de 0,1 à 10 % et de préférence de 0,5 à 3 % du poids total de la composition.

La composition définie ci-dessus est apte à traiter la peau en douceur, c'est-à-dire à améliorer l'éclat du teint, à lisser la peau et à traiter les rides et/ou ridules de la peau. Aussi, la présente invention a encore pour objet l'utilisation cosmétique des compositionsdéfinies ci-dessus pour améliorer l'éclat du teint et/ou pour lisser la peau et/ou traiter les rides et les ridules de la peau.

L'invention a enfin pour objet un procédé cosmétique et/ou dermatologique pour traiter les rides et les ridules de la peau, consistant à appliquer sur la peau ridée les compositions définies ci-dessus.

La composition de l'invention contenant du miel comporte un milieu cosmétiquement et/ou dermatologiquement acceptable, c'est-à-dire compatible avec la peau, le cuir chevelu et les cheveux. Elle peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage ou encore comme produit de maquillage.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80 % en poids, et de préférence de 5 à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées (cyclométhicone) et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG par Shin Etsu), et leurs mélanges.

On peut aussi ajouter des actifs antirides autres que le miel, et notamment des produits tenseurs tels que les protéines végétales et leurs hydrolysats, en particulier l'extrait de protéine de soja vendue sous le nom d'Eleseryl par la société LSN ou le dérivé d'avoine vendu sous la dénomination « Reductine » par la société Silab.

D'autres caractéristiques et avantages de l'invention ressortiront mieux des exemples qui suivent et des figures annexées, donnés à titre illustratif et non limitatif. Les proportions sont données en pourcentage pondéral.

### Exemple 1: Crème pour le visage

### Phase huileuse :

- Huile minérale 15 %
- Alcool cétylique 4 %
- Stéarate de glycéryle 3 %
- Stéarate de PEG-40 2 %
- Tristéarate de sorbitane 0,9 %

### Phase aqueuse :

- Glycérine 3 %
- Miel 2 %
- Eau déminéralisée qsp 100 %

### Mode opératoire :

Les phases aqueuse et huileuse sont chauffées séparément à 70-80°C. Puis la phase huileuse est introduite dans la phase aqueuse à 65°C sous agitation. On refroidit l'émulsion jusqu'à la température ambiante en continuant l'agitation.

On obtient une crème blanche apte à redonner à la peau du visage son éclat.

Afin d'évaluer l'effet kératolytique de cette crème, un test a été réalisé in vivo sur un panel de 18 femmes âgées de 35 à 50 ans ayant la peau sèche. On applique 2 mg/cm² de crème sur la face interne de l'avant-bras de chaque sujet et, 24 heures après application, on compte par cytofluorimétrie les cellules de desquamation libérées. On répète l'opération 4 jours de suite et on fait le compte de toutes les cellules libérées. Le test a été réalisé parallèlement pour une crème identique contenant 5 % d'acides de fruits (acide lactique, acide glycolique, acide citrique, acide malique).

Les résultats montrent que l'effet kératolytique de la crème selon l'invention est équivalent à celui obtenu avec la crème contenant les acides de fruits. En outre, la crème selon l'invention a l'avantage d'être moins irritante que les crèmes contenant des actifs acides, bien qu'aussi efficace.

### Exemple 2 : Fluide pour le visage

### Phase huileuse :

- Huile minérale 5 %
- Alcool cétylique 0,4 %
- Stéarate de glycéryle / stéarate de PEG-100 (Arlacel 165 vendu par la société ICI) 1,6 %

### Phase aqueuse :

- Glycérine 5 %
- Carbomer 0,5 %
- Hydroxyde de sodium 0,2 %
- Miel 2 %
- Eau déminéralisée qsp 100 %

### Mode opératoire :

On fait fondre la phase huileuse à 75°C et on chauffe la phase aqueuse à 80°C. Puis on réalise l'émulsion en introduisant la phase huileuse dans la phase aqueuse à 75°C sous agitation. On refoidit jusqu'à la température ambiante en continuant l'agitation.

L'utilisation quotidienne de ce fluide sur le visage permet de retrouver un teint plus lumineux, une peau plus lisse.

Un test d'efficacité de cette crème a été réalisé sur un panel de 30 femmes. Ces femmes ont appliqué la crème sur le visage une fois par jour pendant cinq jours. 96 % des utilisatrices ont trouvé que la crème était très confortable, même après cinq jours d'application. De plus, après les cinq jours d'utilisation, 69 % des utilisatrices ont trouvé qu'elles avaient meilleure mine, 62 % que leur teint était moins brouillé et 69 % qu'elles avaient une peau plus lisse.

### Exemple 3 : Crème pour le visage

### Phase huileuse :

- Alcool cétylique 4 %
- Huile minérale 15 %
- Stéarate de glycéryle 3 %
- Stéarate de PEG-40 2 %
- Tristéarate de sorbitane 0,9 %

### Phase aqueuse :

- Hydroxyde de sodium 0,4 %
- Glycérine 3 %
- Citrate de sodium (tampon) 1 %
- Acides de fruits (acide lactique, acide glycolique, acide citrique, acide malique) 1 %
- Miel 2 %
- Eau déminéralisée qsp 100 %

Le mode opératoire suivi pour la préparation de cette crème est le même que dans l'exemple 1.

Un test d'efficacité de cette crème a été réalisé sur un panel de 60 femmes ayant la peau sensible. Ces femmes ont appliqué la crème sur le visage une fois par jour pendant cinq semaines. Au bout de ce temps d'application, 78 % des utilisatrices se sont trouvées satisfaites du produit, 47 % ont perçu un effet d'éclat du teint et 56 % ont trouvé qu'elles avaient une peau plus lisse.

Par ailleurs, utilisée en comparaison avec un produit contenant 2 % d'un mélange d'acides de fruits, cette crème a été jugée aussi efficace et moins irritante.

### Exemple 4 : Fluide pour le visage

### Phase huileuse:

- Huile minérale 5 %
- Alcool cétylique 0,4 %
- Stéarate de glycéryle/Stéarate de PEG-100 (Arlacel 165) 1,6 %

### Phase aqueuse:

- Carbomer 0,5 %
- Glycérine 3 %
- Acides de fruits (acide lactique, acide glycolique, acide citrique, acide malique) 1 %
- Hydroxide de sodium 0,4 %
- Tampon citrate 3 %
- Miel 1 %
- Eau déminéralisée qsq 100 %

Le mode opératoire suivi pour la préparation de ce fluide est le même que dans l'exemple 2.

Un test d'efficacité de ce fluide a été réalisé sur un panel de 60 femmes ayant la peau sensible. Ces femmes ont appliqué le fluide sur le visage une fois par jour pendant cinq semaines. Au bout de ce temps d'application, 59 % des utilisatrices se sont trouvées satisfaites du produit, 56 % ont perçu un effet d'éclat du teint et 62 % ont trouvé qu'elles avaient une peau plus lisse.

Par ailleurs, ce fluide a été jugé aussi performant qu'un produit contenant 1,4 % d'un mélange d'acides de fruits, tout en étant moins irritant.

### Exemple 5 : lotion biphase

### Phase huileuse :

- Huile minérale 8,75 %
- Cyclométhicone 10 %
- Huile d'abricot 6,25 %

### Phase aqueuse :

- Glycérine 3,75 %
- Miel 0,75 %
- conservateurs qs
- Eau déminéralisée qsp 100 %

### Mode opératoire :

On chauffe la phase aqueuse à 80°C pour solubiliser les conservateurs ; on mélange puis on refroidit à la température ambiante. On prépare la phase huileuse à température ambiante. On mélange les deux phases et on homogénéise.

On obtient une lotion à deux phases que l'on secoue avant utilisation et qui, au repos, se sépare de nouveau en deux phases. Cette lotion en application quotidienne sur le visage permet de retrouver un teint éclatant.

### Exemple 6 : lotion tonique

- Eau de bleuet 5 %
- D-panthénol 0,2 %
- Miel 0,5 %
- conservateurs qs %
- Eau déminéralisée qsp 100 %

### Mode opératoire :

On chauffe les conservateurs dans l'eau à 80°C pour les solubiliser. Puis, on ajoute les autres constituants. On mélange bien.

On obtient une lotion transparente, aux propriétés tonifiantes et régénérantes.

### Exemple 7 : lotion biphase

### Phase huileuse :

- Huile minérale 10 %
- Cyclométhicone 8,75 %
- Huile de tournesol 6,25 %

### Phase aqueuse :

- Glycérine 6 %
- Acides de fruits (acide lactique, acide glycolique, acide citrique acide malique) 0,375 %
- Hydroxyde de sodium 0,15 %
- Miel 1,5 %
- Tampon citrate 0,75 %
- conservateurs qs
- Eau déminéralisée qsp 100 %

Le mode opératoire suivi pour la préparation de cette lotion est le même que dans l'exemple 5.

### Exemple 8 : lotion tonique

- Allantoïne 0,1 %
- Acides de fruits (acide lactique, acide glycolique, acide citrique, acide malique) 1 %
- Triéthanolamine 0,8 %
- Tampon citrate 3 %
- Miel 1 %
- conservateurs qs
- Eau déminéralisée qsp 100 %

Le mode opératoire suivi pour la préparation de cette lotion est le même que dans l'exemple 6.

## Revendications

1. Utilisation du miel en une quantité efficace dans une composition cosmétique, comme agent kératolytique et/ou comme actif pour améliorer l'éclat du teint et/ou pour lisser la peau et/ou pour traiter les rides et les ridules.

2. Utilisation du miel en une quantité efficace comme agent kératolytique et/ou comme actif pour améliorer l'éclat du teint et/ou pour lisser la peau et/ou pour traiter les rides et les ridules, pour la préparation d'une composition dermatologique.

3. Utilisation selon la revendication 1 ou 2, caractérisée en ce que le miel est composé d'un mélange de glucose, levulose, dextrine, saccharose, protéines et eau.

4. Utilisation selon l'une quelconque des revendications précédentes, caractérisée en ce que le miel ne contient pas d'hydroxyacides.

5. Utilisation selon l'une quelconque des revendications précédentes, caractérisée en ce que le miel est présent en une quantité allant de 0,1 à 10 % du poids total de la composition.

6. Utilisation selon l'une quelconque des revendications précédentes, caractérisée en ce que la composition se présente sous forme d'une lotion, d'un gel aqueux, d'un sérum, d'une émulsion ou d'une dispersion de vésicules lipidiques.

7. Utilisation selon l'une quelconque des revendications précédentes, caractérisée en ce que la composition contient, en outre, au moins un adjuvant choisi parmi les gélifiants, les actifs, les conservateurs, les antioxydants, les solvants, les parfums, les charges, les filtres, les pigments, les agents chélateurs, les absorbeurs d'odeur, les matières colorantes, les agents matifiants et leurs mélanges.

8. Utilisation selon la revendication précédente, caractérisée en ce que l'adjuvant est un actif choisi parmi les α-hydroxy-acides, les β-hydroxy-acides, les α-céto-acides et les β-céto-acides.

9. Utilisation selon l'une quelconque des revendications précédentes, caractérisée en ce que la composition contient, en outre, un alcool gras.

10. Utilisation selon l'une quelconque des revendications précédentes, caractérisée en ce que l'alcool gras est choisi parmi l'alcool cétylique et l'alcool stéarylique.

11. Utilisation selon l'une quelconque des revendications précédentes, caractérisée en ce que la composition contient, en outre, un actif choisi parmi les produits tenseurs.

12. Composition cosmétique ou dermatologique, caractérisée en ce qu'elle contient, dans un milieu cosmétiquement et/ou dermatologiquement acceptable, une quantité efficace de miel et au moins un alcool gras.

13. Composition selon la revendication 12, caractérisée en ce que le miel est présent en une quantité allant de 0,1 à 10 % du poids total de la composition.

14. Composition selon la revendication 12 ou 13, caractérisée en ce que l'alcool gras est présent en une quantité allant de 0,1 à 10 % du poids total de la composition.

15. Composition selon l'une quelconque des revendications 12 à 14, caractérisée en ce qu'elle contient en outre un actif acide.

16. Composition cosmétique ou dermatologique, contenant dans un milieu cosmétiquement et/ou dermatologiquement acceptable, au moins un actif acide, caractérisée en ce qu'elle contient, en outre, du miel et un tampon de pH 5.

17. Composition selon la revendication précédente, caractérisée en ce que l'actif acide est choisi parmi les α-hydroxy-acides, les β-hydroxy-acides, les α-céto-acides et les β-céto-acides.

18. Composition selon la revendication 16 ou 17, caractérisée en ce que l'actif acide est présent en une quantité allant de 0,01 à 20 % du poids total de la composition.

19. Composition selon l'une quelconque des revendications 16 à 18, caractérisée en ce que le tampon est un tampon citrate.

20. Composition selon l'une quelconque des revendications 16 à 19, caractérisée en ce que le tampon est présent en une quantité allant de 0,1 à 10 % du poids total de la composition.

21. Composition selon l'une quelconque des revendications 12 à 20, caractérisée en ce qu'elle se présente sous forme d'une lotion, d'un gel aqueux, d'un sérum, d'une émulsion ou d'une dispersion de vésicules lipidiques.

22. Composition selon l'une quelconque des revendications 12 à 21, caractérisée en ce que la composition contient, en outre, au moins un adjuvant choisi parmi les gélifiants, les actifs autres que l'actif acide, les conservateurs, les antioxydants, les solvants, les parfums, les charges, les filtres, les pigments, les agents chélateurs, les absorbeurs d'odeur, les matières colorantes, les agents matifiants et leurs mélanges.

23. Composition selon l'une quelconque des revendications 12 à 22, caractérisée en ce que la composition contient, en outre, un actif choisi parmi les produits tenseurs.

24. Utilisation cosmétique de la composition selon l'une quelconque des revendications 12 à 23, pour améliorer l'éclat du teint et/ou pour lisser la peau et/ou traiter les rides et les ridules de la peau.

25. Procédé cosmétique pour traiter les rides et les ridules de la peau, consistant à appliquer sur la peau ridée la composition selon l'une quelconque des revendications 12 à 23.
